Europäisches Patentamt

(19) European Patent Office

Office européen des brevets

(11) Publication number : 0 522 825 A1

# (12) EUROPEAN PATENT APPLICATION

(21) Application number : 92306215.2

(22) Date of filing : 07.07.92

(51) Int. Cl.$^5$ : **A61F 13/04, A41D 19/00**

(30) Priority : 08.07.91 US 726468

(43) Date of publication of application :
13.01.93 Bulletin 93/02

(84) Designated Contracting States :
AT BE CH DE ES GB IT LI LU NL

(71) Applicant : JOHNSON & JOHNSON
ORTHOPAEDICS, INC.
325 Paramount Drive
Raynham, Massachusetts 02767-0350 (US)

(72) Inventor : Green, Richard
120 East Cedar Street
Livingston, NJ 07039 (US)
Inventor : Oser, Zale
87 Pastureview Lane
Cranston, RI 02921 (US)

(74) Representative : Mercer, Christopher Paul et al
Carpmaels & Ransford 43, Bloomsbury Square
London WC1A 2RA (GB)

(54) **Gloves for applying an orthopaedic bandage.**

(57) A glove (13) for applying resin coated orthopaedic casting bandage material (12) is provided which glove is formed of an elastomeric layer made of for example, butyl rubber, latex or polyurethane. The elastomeric layer defines an inner surface and an outer surface which together define an open cuff, hand portion and finger portions. On the outer surface of the elastomeric layer is provided a substantially dry coating (14) which coating becomes lubricous when wet (15). The coating may be a polyvinyl pyrollidone-polyurethane interpolymer.

FIG-5

EP 0 522 825 A1

## Field of the Invention

The present invention relates to an improved glove for applying orthopaedic casting bandages of the type used to form orthopaedic casts. In particular the invention relates to a glove for applying orthopaedic bandages of the type having an uncured resin coating thereon.

## Background of the Invention

Plaster of paris casts have been used to immobilize body members for some time. These bandages are made by depositing plaster of paris on a reinforcing scrim material such as gauze. When the plaster of paris is dipped in water, reactions take place which result in the hardening of the cast material. Plaster of paris casts, however, suffer from a number of disadvantages. X-ray transmission through the cast to determine whether a fracture has properly set is extremely difficult. In addition, the cast is quite heavy and restricts the mobility of patients wearing the cast.

In order to overcome the disadvantages of plaster of paris casts, numerous attempts have been made to develop plastic or plastic reinforced materials as replacements for plaster of paris. U.S. patent numbers 3,241,501 and 3,881,473 disclose casts which are made with a flexible fabric impregnated with a polymer which is capable of being cured by ultraviolet light.

Other attempts to replace plaster of paris casts are disclosed in German Offenlegenschrift numbers 2353212 and 2357931, U.K. Patent #1,578,895, and PCT Application #WO 81/00671. These bandages are open weave fabrics coated with polyurethane prepolymers, that is, reaction products of isocyanates and polyols. The bandages are dipped into water in the same manner as the plaster of paris and then applied to the limb of the patient. The water causes the prepolymer to polymerize and form a rigid polymer structure.

More recently it has been found that in working with such materials having a prepolymer resin coating that the tackiness of the resin of the bandage can make working with the bandages difficult and cumbersome for the doctor. In an attempt to address this issue a glove lubricant comprised of water, sorbitol, mineral oil and silicon fluid has been sold by 3M Company, St. Paul, Minn., under the trade name CastCreme with instructions to apply the lubricant to the gloves of one applying an isocyanate-functional prepolymer coated cast after wrapping of the cast but before molding of the cast to avoid having the exposed casting material adhere to the gloves of the one applying the cast. This is disclosed in the background of U.S. Patent 4,667,661 and 4,774,937.

The '661 and '937 patents are directed to addressing the adherence issue by providing the resin itself with a lubricant. The curable resin coated sheet is prelubricated with a lubricant which is either a) bonded to the resin, b) added to the resin or applied to the surface of the coated sheet or c) provided in a combination of the bonding and surface application described. In many instances however, the tacky feature of the orthopaedic bandage is desirable. As by way of example when the applier is attempting to get the end of the bandage to stick to the surface of the bandage wrap in order to terminate the application of the bandage. The addition of lubricant in the resin permits relative slipping of the resin coated sheet and requires molding the cast in position and holding it in position to prevent slippage.

Coatings for substrates having a low coefficient of friction have been shown in U.S. patent 4,100,309 entitled "Coated Substrate Having a Low Coefficient of Friction Hydrophilic Coating and a Method of Making the Same". That reference describes a substrate which is coated with a polyvinyl pyrollidone-polyurethane inter polymer. In the method, a poly-isocyanate and a polyurethane in a solvent such as methyl ethyl ketone are applied to a substrate and the solvent evaporated. If the substrate is a polyurethane, only the polyisocyanate need be employed. Polyvinyl pyrollidone in a solvent is then applied to the treated substrate and the solvent evaporated. The substance and coated objects described in this reference are used in blood and body contacting environments. In order to lubricate the introduction of devices into openings in the body.

## Summary of the Invention

The invention comprises a glove for applying resin coated orthopaedic material which glove comprises an elastomeric layer having an inner surface and an outer surface which define an open cuff, a hand portion and finger portions. The glove is provided with a substantially dry coating on the outer surface which becomes lubricous when wetted. In particular the coating may be a non-water based solvent coating. For example the coating may be in part polyvinyl pyrollidone. The flexible layer of the glove may be made of other materials such as butyl rubber, or latex. Also in keeping with the invention the coating may be a water-based solution which is applied to the glove and permitted to dry.

In a preferred embodiment the glove may have an elastomeric layer on which the coating is applied which coating comprises a polyvinyl pyrollidone-polyurethane interpolymer. The glove material may be an elastomeric layer made of polyurethane.

## Brief Description of the Drawings

The invention will now be described with reference to the accompanying drawings wherein:
Fig. 1 is a perspective view of a package containing the materials for use in the present invention;
Fig. 2 shows a package containing a resin coated substrate for use as an orthopaedic bandage;
Fig. 3 shows the package of Fig. 2 being opened using the gloves of the present invention;
Fig. 4 depicts the glove of the present invention used in a method of applying an orthopaedic bandage;
Fig. 5 shows the immersion of the orthopaedic material by the glove covered hand in a bath to activate the material;
Fig. 6 shows wrapping of the material about the limb of a patient in order to apply the material thereto.

## Detailed Description of the Invention

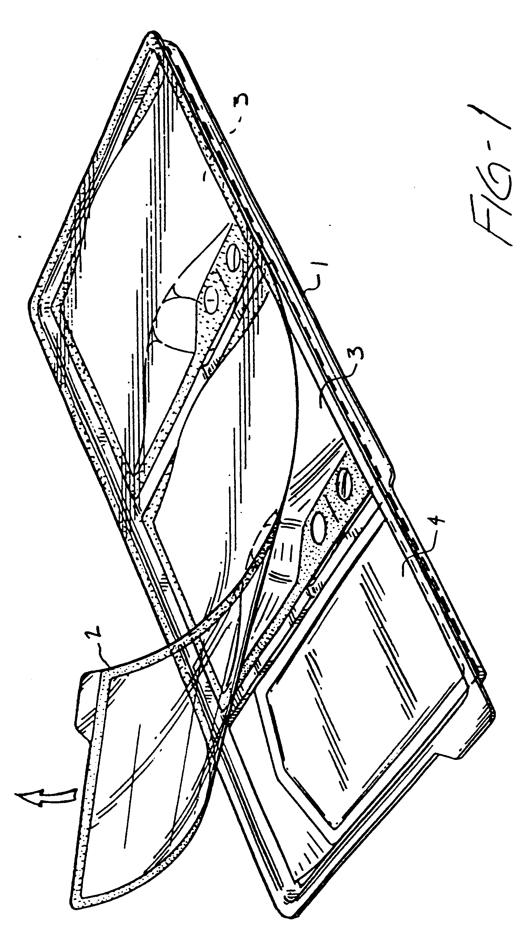

Figure 1 depicts a kit-like structure in which the supplies for using the gloves of the present invention are provided. In particular a shell (1) having a peelable layer (2) defining a sealed inner volume is provided. The shell may be formed so as to provide as many compartments as necessary for providing the kit. In particular as shown in Figure 1 compartments (3) are provided for containing orthopaedic bandage material and compartment (4) is provided in order to supply at least one pair of coated gloves of the invention.

In use the package is opened by peeling peelable layer (2) away from shell (1) exposing the packages of orthopaedic bandage and gloves. The package (5) is removed and contains a roll of predetermined length of orthopaedic bandage material having uncured prepolymer resin deposited thereon. The bandage may be, for example, a knitted fiberglass substrate having a polyurethane prepolymer thereon such as the bandage sold by Johnson & Johnson Orthopaedics under the trade name Delta-Lite® fiberglass casting tape. The package (5) is formed of a top sheet (6) and bottom sheet (7) which are sealed about their periphery by, for example, heat sealing to form a unitary package. Sealed portion (8) widens at one corner to provide space to define openings (9) which are positioned on opposite sides of slit (10).

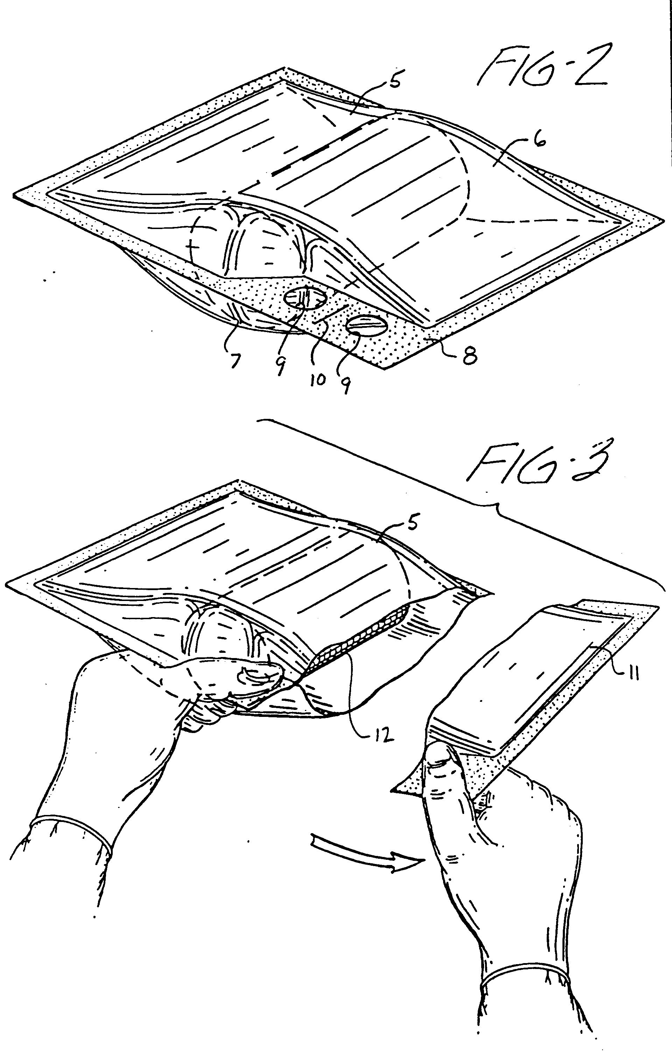

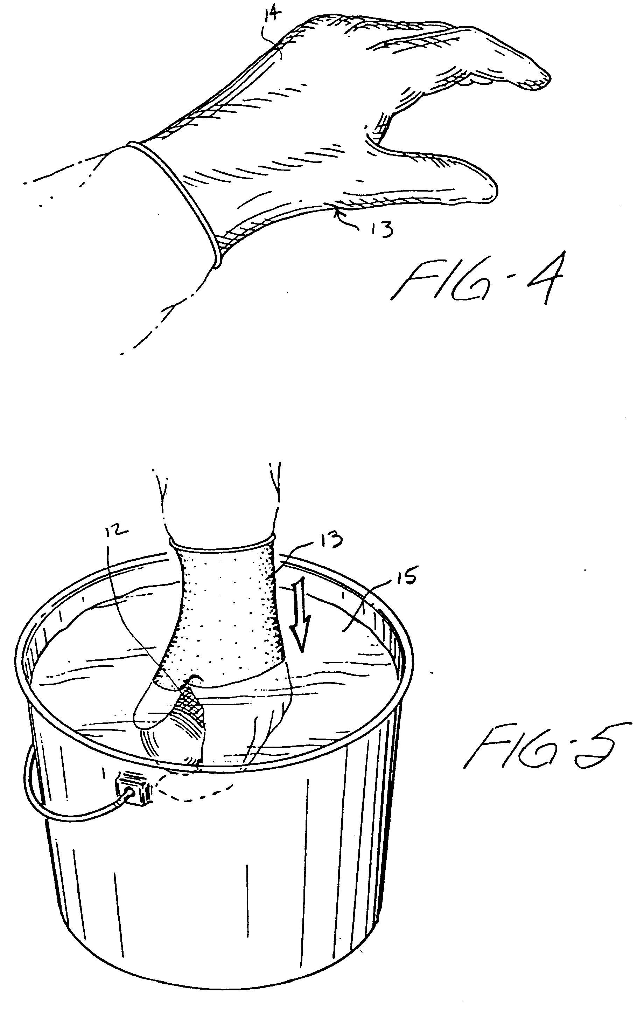

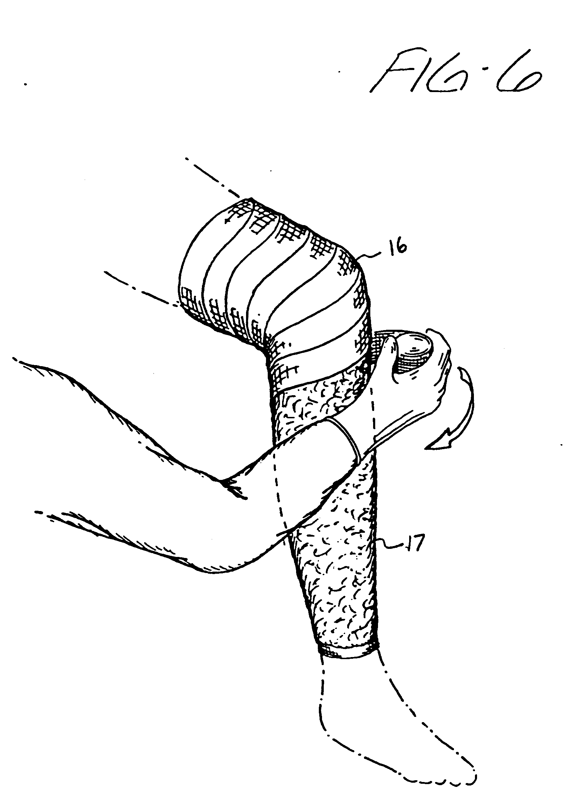

In use the package may be opened during the application of the orthopaedic bandage to the limb of the patient. In such a situation the applier would already be wearing the gloves of the invention, which will be described below, in order to provide a lubricous contact between the hands of the applier and the orthopaedic bandage material. In order to facilitate the opening of the package, openings (9) permit points at which the applier may grasp the package either by insertion of a finger or by contact of the fingers in a gripping fashion through the opening so that the hands are not slipping on the outer surface of the package. Slit (10) provides a start for the opening tear of the package as well as a stress point- to facilitate opening of the package. A portion of the package is torn away as shown in Figure 3 and waste section (11) is discarded.

Thus opened the applier is provided access to a roll (12) of orthopaedic bandage material having thereon a prepolymer coating.

Glove (13) has an outer surface (14) having thereon a substantially dry coating which forms a lubricous surface when wetted during the application of the bandage as is described below. This glove may be formed of any suitable substance such as for example butyl rubber, latex, polyvinyl chloride polyvinyl alcohol, neoprene or other natural or synthetic polymeric materials.

The coating is applied to the glove substantially as described in U.S. patent 4, 100,309. The coating may be, for example, polyvinyl pyrollidone which is applied in a non-water based solvent carrier as follows. Preformed latex rubber gloves are mounted in multiple clamping devices on a rack where the gloves are pressurized with air in order to partially inflate them and fully expose the gloves' outside surfaces. The rack is lowered into position so that the inflated gloves dip into a container of the solvent carrier solution for a period of time sufficient to completely wet the outside surfaces of the gloves. The rack, with attached gloves, is removed from its position above the dip bath and placed in an oven where solvent is allowed to evaporate and the resultant coating on the gloves is heated to facilitate curing.

The coating may also be provided in a water based fashion as follows. A machine for the continuous manufacture of latex rubber gloves is equipped with an additional dip tank positioned at the end of the line so that the latex gloves, prior to final drying, are subjected to an overdip of an aqueous based formulation in order to provide the coating to the glove, described above.

The coating applied to the gloves as an overdip at the end of the latex glove manufacturing line is applied from an aqueous bath whose solids (non-aqueous) components make up 14% of the total bath. These components are:

| Component | % of Total |
|---|---|
| 2-Pyrol (GAF) | 78.5 |
| Desmodur XP7005 (Mobay) | 1.8 |
| Glycerine (Dial) | 2.3 |
| Igepal CO-630 (Rhone Polenc) | 3.6 |
| Polyvinyl Pyrrollidone K-90 (GAF) | 11.5 |
| Kelco K7C233 (Kelco) | 1.4 |
| Impranil DLN (Mobay) | 0.4 |
| Neorez R-962 (ICI) | 0.5 |
| | 100.0 |

If a distinctively colored glove is desired, a pigment may be added to the above overdip bath or to the bath preceding the overdip bath on the production line. For example, if a light blue colored glove is desired one can add 0.02% Stan-Tone® 40WD01 Blue (Harwick Chemical Corp) to the latex substrate. This results in a finished glove with an attractive blue color, after the overdip coating has been accomplished.

Over Dip Coating Composition of Solids in Aqueous Systems

1. 2-Pyrol (GAF) - 2-pyrrolidone
2. Desmodur XP7005 (Mobay) - Blocked Aromatic Isocyanate Prepolymer
3. Glycerine (Dial Corp) - Glycerine
4. Igepal CO-630 (Rhone Polenc) - Surfactant Nonylphenyxy poly(ethyleneoxy) ethanol
5. PVP/K-90 (GAF) - Polyvinylpyrollidone
6. Kelco K7C233 (Kelco) - Sodium alginate (Hydrophilic colloid)
7. Impranil DLN (Mobay) - Anionic aqueous dispersion of an aliphatic polyester polyurethane
8. Neorez R-962 (ICI) - Aqueous dispersion of an aliphatic polyurethane

Upon donning glove (13) and opening the package as described above, access is gained to the orthopaedic bandage material. The orthopaedic bandage material is immersed in a bath (15) which may conveniently be water if a prepolymer is used which is activatable by immersing in water. The coating on the surface (14) of glove (13) is also in a manner activated by the water of the bath. The surface is preferred to be treated such that when wetted by the bath it becomes significantly more lubricous than it was prior to being wetted. This lubricousness relative to the resin impregnated orthopaedic bandage permits the application of the bandage (16) over a stockinette (17) in a known manner. The bandage (16) adheres to itself in order to maintain its laminable position while at the same time being handled by a glove (13) having a lubricous surface which permits an easily slidable handling of the orthopaedic material by the glove covered hand.

## Claims

1. A glove for applying resin coated orthopaedic material comprising:
   a) an elastomeric layer having an inner surface and an outer surface defining an open cuff, a hand portion and finger portions; and
   b) a substantially dry coating on said outer surface which coating is lubricous when wet.

2. The glove according to claim 1 wherein:
   c) said glove with said coating is more lubricous relative to resin coatings of uncured polyurethane than said glove absent said coating; and
   d) said coating is hydrophilic.

3. The glove according to claim 1 or claim 2 wherein the elastomeric layer is formed of latex.

4.   The glove according to claim 1 or claim 2 wherein the elastomeric layer is polyurethane.

5.   The glove according to any one of claims 1 to 4 wherein the coating is a non-water based solvent coating.

6.   The glove according to any one of claims 1 to 4 wherein the coating is applied in a solution with water.

7.   The glove according to any one of claims 1 to 6 wherein the coating comprises polyvinylpyrollidone.

8.   The glove according to any one of claims 1 to 7 wherein the coating is a polyvinyl pyrollidone-polyurethane interpolymer.

FIG-1

FIG·2

FIG·3

FIG·4

FIG·5

FIG·6

16

17

**European Patent Office**

## EUROPEAN SEARCH REPORT

Application Number

EP 92 30 6215

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl.5 ) |
| X<br>Y | US-A-4 589 873 (A.SCHWARTZ ET AL.)<br>* column 1, line 56 - line 59 *<br>* column 2, line 33 - line 68 *<br>* column 3, line 29 - line 37 *<br>--- | 1-5,7-8<br>6 | A61F13/04<br>A41D19/00 |
| Y | FR-A-2 315 382 (SUTURES)<br>* page 1, line 7 - line 15 *<br>* page 1, line 39 - page 2, line 1 *<br>* page 3, line 16 - line 22 *<br>* page 4, line 32 - line 39 *<br>* page 6, line 28 - line 31 *<br>--- | 6 | |
| D,A | US-A-4 100 309 (M.J.MICKLUS AND D.T.OU-YANG)<br>* column 1, line 8 - line 15 *<br>* column 1, line 28 - line 35 *<br>* column 1, line 63 - column 2, line 3 *<br>--- | 1-5,7-8 | |
| D,A | US-A-4 774 937 (M.T.SCHOLZ ET AL.)<br>* column 2, line 39 - line 46 *<br>* column 6, line 54 - line 62 *<br>* column 11, line 23 - line 31 *<br>----- | 1-2 | TECHNICAL FIELDS SEARCHED (Int. Cl.5 )<br><br>A61F<br>A61B<br>A41D |

The present search report has been drawn up for all claims

| Place of search | Date of completion of the search | Examiner |
|---|---|---|
| THE HAGUE | 09 OCTOBER 1992 | NICE P. |

EPO FORM 1503 03.82 (P0401)